# EUROPEAN PATENT APPLICATION

(11) **EP 4 420 688 A1**
(43) Date of publication of application: **28.08.2024**
(21) Application number: 23158008.5
(22) Date of filing: 22.02.2023
(51) Int. Cl.: A61L 2/22, B01F 23/00, B01F 33/84, B05B 17/06

(54) **LIQUID DISPENSER**

(71) Applicant: Fast & Fluid Management B.V., 2171 KZ Sassenheim (NL)
(72) Inventor: Baak, Mark, 2161 TM Lisse (NL)
(74) Representative: De Vries & Metman

(57) **Abstract**

Dispenser, e.g., for tinting pastes, comprising at least one container with a lid. The lid comprises a compartment for holding a liquid, and an atomizer, such as an ultrasonic fogger, arranged to atomize the liquid from the compartment into a headspace of the container. The liquid can for example be an aqueous solution of a disinfectant.

## Description

The present disclosure relates to a liquid dispenser comprising at least one container for holding a liquid to be dispensed, such as a water borne emulsion or dispersion, in particular a pigment dispersion, e.g., a paint colorant or tinting paste. Such dispensers can be used to dispense and mix selected colorants or tinting pastes, optionally with a selected base paint, in accordance with a pre-defined formulation of a desired paint colour, for instance at a point of sale or a car refinish body shop.

Due to environmental regulations, present day paints and tinting pastes are usually water borne. Such aqueous compositions are sensitive for microbiological activity, in particular growth of mould, algae, bacteria or other microorganisms. This is particularly problematic for tinting pastes, which are usually stored for longer periods in containers where only occasionally small amounts are dispensed from. Microbiological fouling and mildew can for example occur in the headspace of such containers or at the dispensing nozzle.

EP 3 702 320 A1 discloses a dispenser with tinting paste containers having a lid with a nozzle arranged to spray additives, such as disinfectants into the headspace of the respective container.

It is an object of the invention to provide a simpler dispenser allowing disinfection of canister headspaces. It is a further object to provide a system providing a retrofit solution for existing dispensers.

The object of the invention is achieved with a dispenser comprising at least one container with a lid, the lid comprising a compartment for holding or storing a liquid, and an atomizer, such as a sprayer or fogger or nebulizer arranged to atomize the liquid from the compartment into the headspace of the container.

In this respect, a sprayer is an atomizer generating a dispersion of droplets of a larger average droplet size, e.g., of at least 60 micrometer, typically moving in a substantially straight spraying direction. A fogger or nebulizer generates a fog or mist of smaller droplets, typically moving with the flow of the surrounding air. Such a fog is more evenly distributed into the headspace than a spray and significantly improves wetting of the interior walls.

The atomizer can for instance be an ultrasonic fogger or nebulizer. An ultrasonic fogger comprises an ultrasonic transducer plate in a liquid reservoir. When powered and activated, the ultrasonic transducer vibrates at an ultrasonic frequency and causes water to break apart into aerosols which vaporize to form a dense fog. Ultrasonic foggers are compact and can easily be integrated in a container lid.

Optionally, the atomized liquid comprises one or more additives such as disinfectants. Suitable disinfectants are for example aqueous solutions of sodium pyrithione.

Other types of additives, such as co-solvents, anti-settling agents and/or rheological agents can also be added to the liquid to be atomized, if so desired.

The lid can for example comprise a refill opening for filling the compartment with the liquid. Alternatively, the lid can comprise a receiving cavity for receiving a replaceable cartridge or capsule containing the liquid.

The dispenser may comprise a control unit to activate the atomizer, e.g., to generate a fog in the headspace at predefined intervals and/or in response to a sensor. The control unit can be the main control unit of the dispenser, or can be a separate control unit, e.g., a programmable control unit within the lid. The control unit can communicate with the atomizer, e.g., via a wireless transmission, such as BlueTooth^{®}. The control unit can also be used to monitor liquid level within the liquid reservoir, and/or to monitor battery charge level.

Optionally, a humidity sensor can be used to monitor relative humidity in the headspace. In response to the sensor output, the control unit can trigger the atomizer if the measured humidity gets below a preset threshold value.

In a specific embodiment, the lid can comprise a sensor to detect whether or not the lid is put on a container. The control unit can for example be configured to activate the atomizer to generate a fog or spray every time the lid is temporarily removed and replaced to re-humidify and disinfect the headspace.

The lid may further comprise one or more batteries for powering the atomizer and circuitry connecting the batteries to the atomizer.

Optionally, the atomizer can comprise a user interface to provide information to an operator, such as liquid level or battery level, and/or an input section for changing settings, such as timing intervals for atomizing.

In a specific embodiment, the dispenser can comprise a plurality of containers and a support for carrying the containers, such as a turntable or a static platform. Each container comprises a dispense nozzle, e.g., at a bottom side of the container. The dispenser comprises at least one pump connected to, or selectively connectable to a respective one of the containers. More specifically, each container can be provided with a pump, or the containers can selectively be connected to a central pump. Each pump is provided with a valve movable between a position allowing withdrawal of liquid from the container by the pump, and a second position closing off the container and allowing dispensing of withdrawn liquid from the pump. Each of the valves and pumps is controlled by a control unit to dispense selected amounts of the respective tinting paste in accordance with a paint formulation of a selected colour.

Optionally, the containers are provided with a stirrer, e.g., an upright rotor with blades.

The dispenser is typically suitable for use for dispensing and mixing colorants, in particular water-borne tinting pastes for paints, e.g., at a point of sale.

The invention is further explained with reference to the accompanying drawings showing exemplary embodiments.
Figure 1A: shows a dispenser in perspective view;
Figure 1B: shows the dispenser of Figure 1A without a cover lid;
Figure 2: shows a container of the dispenser of Figure 1 in exploded view;
Figure 3A: shows a lid of the container of Figure 2 in exploded view;
Figure 3B: shows the lid of Figure3A from a lower view point;
Figure 4: shows an alternative container of the dispenser of Figure 1 in exploded view;
Figure 5A: shows a lid of the container of Figure 4 in exploded view;
Figure 5B: shows the lid of Figure 5A from a lower view point.

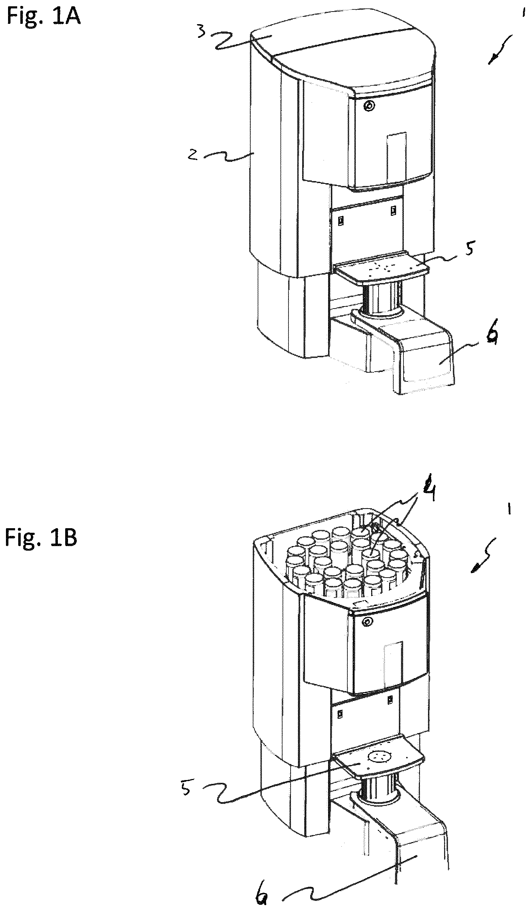

Figures 1A and 1B show a dispenser 1 for mixing and dispensing paint products. The dispenser 1 has a dispenser housing 2 with a cover lid 3. Figure 1B shows the dispenser 1 without the cover lid 3, in order to show the internals of the dispenser 1. The internals of the dispenser 1 encase containers or canisters 4 arranged in concentric circular arrays. Each container 4 holds a water borne tinting paste of a specific colour. The containers 4 are placed in an upright position on a turntable (not shown) which is coaxial with the circular arrays of containers 4. The dispenser 1 further comprises a platform 5 for positioning a paint can or similar receptacle (not shown).

The dispenser 1 comprises a control unit and a user interface 6, such as a touch screen, in communication with the control unit enabling an operator or user to input a desired paint colour. When a user inputs a desired paint colour, the control unit determines a paint formulation matching the selected paint colour, e.g., by selecting the paint formulation from a database. The determined paint formulation consists of a tinting paste or of a mixture of tinting pastes available in the respective containers 4 of the dispenser 1, and optionally a base paint. The control unit controls rotation of the turntable to sequentially position the containers 4 containing the required tinting pastes above the paint can on the platform 5 and control dispensing of the tinting pastes one by one in accordance with the paint formulation.

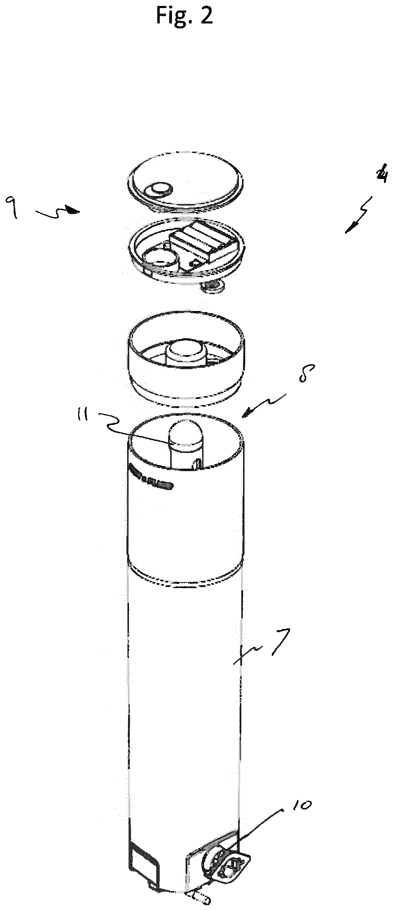

Figure 2 shows a container 4 in exploded view. The container 4 has a cylindrical body 7 with an open top end 8. Each container 4 has a lid 9 closing off the open top end 8. The container 4 further has an outlet port 10 near the bottom of the container 4. The outlet port 10 can be connected to a pump via a valve, to allow withdrawal of a metered amount of liquid from the container by means of the pump, e.g., a piston pump (not shown).

The container 4 encases a stirrer rotor 11 to prevent settling of the contained liquid.

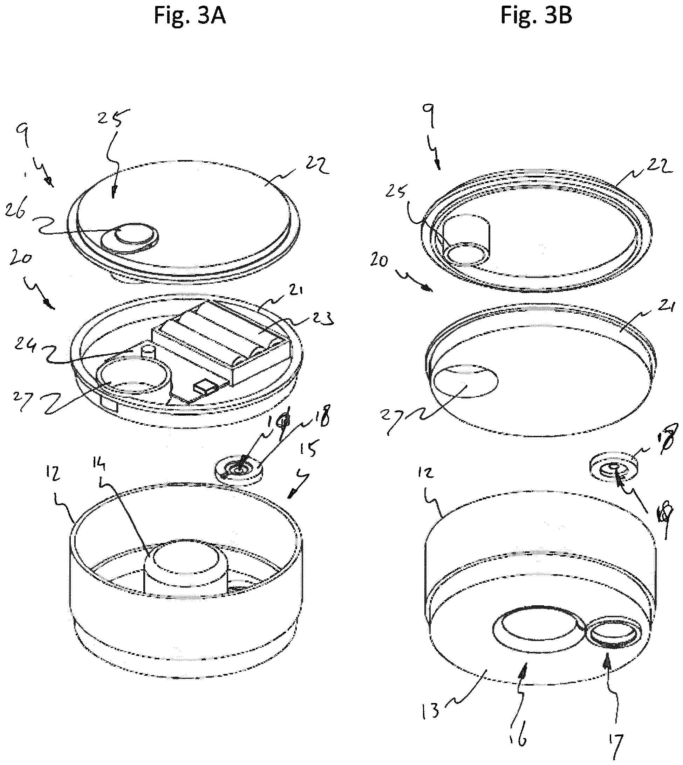

Figures 3A and 3B show the lid 9 in exploded view in more detail from two different viewpoints. The lid 9 comprises a cylindrical base 12 with a bottom 13, a cylindrical wall 14 attached to the bottom 13 and an open top end 15. The bottom 13 has an outlet opening 17 and a central cylindrical recess 16 receiving a top end of the stirrer rotor 11. An ultrasonic transducer element 18 with a central nozzle opening 19 is received in the outlet opening 17 of the bottom 11.

The cylindrical base 12 forms a liquid reservoir closed by a cover 20 with a lower cover half 21 and an upper cover half 22. The upper and lower cover halves 21, 22 enclose an inner space with batteries 23 and circuitry 24 for driving the ultrasonic transducer element 18. The upper cover half 22 has a refill opening 25 with a releasable cap 26. The refill opening 25 communicates with the interior space of the cylindrical base 12 via a passage 27 in the lower cover half 21.

The interior space of the lid 9 can be filled with a liquid that can be nebulized, typically water, optionally with disinfectants and/or other additives. A control unit is configured to activate the ultrasonic transducer element 18 to vibrate at an ultrasonic frequency causing the liquid to form aerosols which vaporize to form a dense fog, which leaves the lid 9 via the opening 17 to enter the headspace of the canister 4. The control unit communicates with the ultrasonic transducer element 18, via a wireless transmission, such as BlueTooth^{®}. Alternatively, the lid 9 itself can be provided with a programmable control unit.

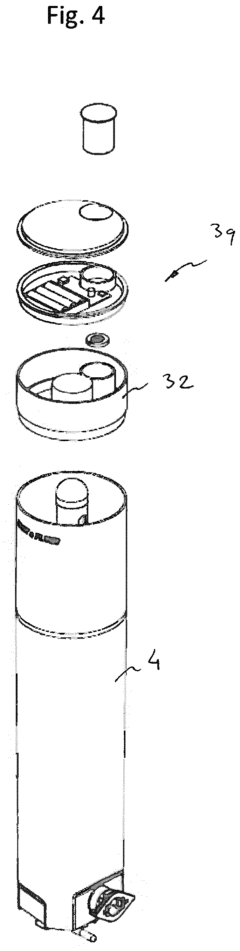

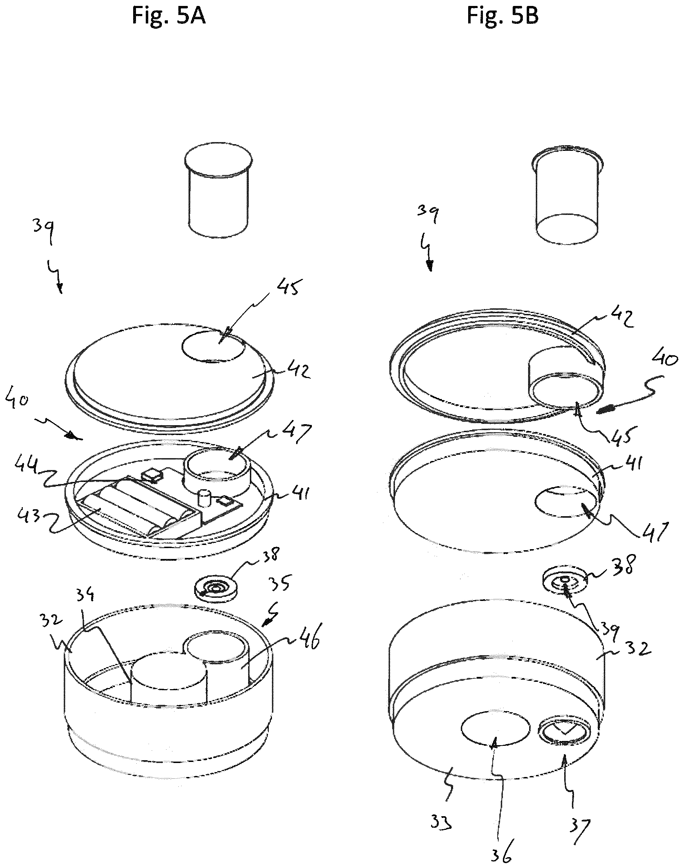

Figure 4 shows an alternative embodiment of a lid 39 for a canister 4. Figures 5A and 5B show the lid 39 in exploded view in more detail. Like the lid 9 of Figure 3, the lid 39 comprises a cylindrical base 32 with a bottom 33, a cylindrical wall 34 attached to the bottom 33 and an open top end 35. The bottom 33 has an outlet opening 37 and a central cylindrical recess 36 receiving a top end of the stirrer rotor 11. The outlet opening 37 is surrounded by a cylindrical wall 46. An ultrasonic transducer element 38 with a central nozzle opening 39 is received in the outlet opening 37 of the bottom 33.

The cylindrical base 32 is closed by a cover 40 with a lower cover half 41 and an upper cover half 42. The upper and lower cover halves 41, 42 enclose an inner space with batteries 43 and circuitry 44 for driving the ultrasonic transducer element 38. The upper cover half 42 has an opening 45 in line with a passage 47 in the lower cover half 21 and in line with the cylindrical wall 46 in the cylindrical base 32. The opening 45 in the upper cover half 42, the passage 47 in the lower cover half 41 and the cylindrical wall 46 define a cavity receiving an inserted replaceable cartridge or capsule 48 with a sliding fit. The cartridge 48 contains the liquid to be nebulized and can be replaced with a new cartridge 48 after the liquid has been completely used up. The cartridge 48 is pierced upon insertion, allowing the contained liquid to contact the ultrasonic transducer element 38.

The control unit can activate the ultrasonic transducer element 38 to vibrate at an ultrasonic frequency causing the liquid in the cartridge 48 to form a dense fog, which leaves the lid 39 via the opening 37 to enter the headspace of the canister 4.

## Claims

1. Dispenser comprising at least one container with a lid, the lid comprising:
- a compartment for holding a liquid and
- an atomizer, such as a sprayer or fogger, arranged to atomize said liquid from the compartment into a headspace of the container.

2. Dispenser according to claim 1, wherein the atomizer comprises a fogger, e.g., an ultrasonic fogger.

3. Dispenser according to claim 1 or 2, the liquid comprising an additive, such as a disinfectant.

4. Dispenser according to claim 3, wherein the compartment comprises a refill opening.

5. Dispenser according to claim 3 or 4, wherein the compartment is a replaceable part of the lid.

6. Dispenser according to any one of the preceding claims, arranged to spray the liquid into the headspace at predefined intervals.

7. Dispenser according to any one of the preceding claims comprising a sensor to check if the lid is put on a container.

8. Dispenser according to any one of the preceding claims, wherein the lid comprises at least one battery powering the sprayer.

9. Dispenser according to any one of the preceding claims, the sprayer comprising a user interface to provide information to an operator, such as liquid level or battery level, and/or an input section for changing settings, such as timing intervals for fogging.

10. Cartridge forming a replaceable compartment of a fogger for a dispenser according to claim 4.
